# EUROPEAN PATENT APPLICATION

(11) **EP 3 753 556 A1**
(43) Date of publication of application: **23.12.2020**
(21) Application number: 19181234.6
(22) Date of filing: 19.06.2019
(51) Int. Cl.: A61K 31/05, A61K 31/352, A61K 36/185, A61K 9/00, A24B 13/00, A24B 15/00, A24D 1/00, A61P 25/04, A61P 25/18, A61P 25/20, A61P 25/24, A61P 25/30, A61P 25/34, A61P 25/36, A61K 9/72

(54) **COMPOSITION AND SMOKABLE PRODUCT COMPRISING CANNABIDIOL FOR USE IN THE TREATMENT OF MENTAL DISORDER AND/OR DRUG ADDICTION AND/OR SLEEP DISORDER AND/OR PAIN**

(71) Applicant: Universität Basel, 4003 Basel (CH)
(72) Inventor: Lang, Prof. Dr. Undine, 4055 Basel (CH); Lang, Dr. Elisabeth, 4056 Basel (CH)
(74) Representative: Patentanwälte Ruff, Wilhelm, Beier, Dauster & Partner mbB

(57) **Abstract**

The invention relates to a composition for use in the treatment, in particular symptomatic treatment, of
- mental disorder, preferably psychosis and/or depression,
and/or
- drug addiction, preferably nicotine addiction and/or tetrahydrocannabinol addiction,
and/or
- sleep disorder
and/or
- pain,
wherein the composition comprises tobacco and cannabidiol or tobacco and cannabis comprising cannabidiol and the composition is prepared for an inhalative administration.

Further, the invention relates to a smokable product for use in the treatment, in particular symptomatic treatment, of
- mental disorder, preferably psychosis and/or depression,
and/or
- drug addiction, preferably nicotine addiction and/or tetrahydrocannabinol addiction,
and/or
- sleep disorder
and/or
- pain,
wherein the smokable product comprises or consists of the composition.

## Description

### FIELD OF THE INVENTION

The present invention relates to a composition, in particular medical or pharmaceutical composition, for use in the treatment, in particular symptomatic treatment, of mental disorder, preferably psychosis and/or depression, and/or drug addiction, preferably nicotine addiction and/or tetrahydrocannabinol (THC) addiction, and/or sleep disorder and/or pain.

Psychosis is a mental disorder which affects about 1% of the population. About 50% of the patients are treated against their will with neuroleptic drugs such as antidopaminerg acting substances resulting in unfavorable side effects such as akathisia, parkinsonism and depression. Nearly half of the patients who have been hospitalized interrupt their treatment within a year, since they typically have no understanding of the disease and do not realize that they have a disease. Thus, often enforcement measures are needed to treat the patients, but in the long run result in a low compliance. Altogether, psychosis is one of the most common reasons for early rental dependency of young men and is the one psychiatric disease occurring with increased rates of suicides, aggressive incidents, decreased life expectancy (reduced up to 10-15 years) and reduced therapeutic treatment success.

It is known that cannabis contains various cannabinoids exhibiting pharmacological properties. Specifically, it is known that tetrahydrocannabinol (THC) acts as a psychotomimetic compound, while cannabidiol exhibits antipsychotic properties. In particular, it has been found that cannabidiol is able to antagonize the effects of THC (Zuardi et al.: "A Critical Review of the Antipsychotic Effects of Cannabidiol: 30 Years of a Translational Investigation", Current Pharmaceutical Design, 2012, 18, 5131-5140 and Morgan et al.: "Effects of cannabidiol on schizophrenia-like symptoms in people who use cannabis", The British Journal of Psychiatry, 2008, 192, 306-307).

Tablets containing cannabidiol for treating psychosis and anxiety disorders are known from EP 3 001 812 B1.

Since patients suffering from psychosis, in particular schizophrenia, are usually very suspicious, in particular with respect to treatment methods and medicinal staff, conventional treatment of psychosis is often accompanied by low or decreasing compliance and undesired treatment interruptions. This, in turn, can seriously lower the treatment quality, thereby endangering the treatment success.

### OBJECT AND SOLUTION

In view of the foregoing, an object underlying the present invention is therefore to make available compositions and products for use in the treatment of mental disorder, in particular psychosis and/or depression, which at least partly circumvent the drawbacks mentioned above in the context of conventional treatment of psychosis, in particular which avoid or at least reduce unfavorable side effects, reduce coercive treatment and increase compliance.

Further, it is an object of the present invention to make available products for use in the treatment of drug addiction, in particular nicotine addiction and/or tetrahydrocannabinol (THC) addiction, and/or sleep disorder and/or pain.

The above objects are accomplished by a composition according to claim 1 and a smokable product according to claim 14. Preferred embodiments are defined in the dependent claims and the present description. The subject-matter and wording, respectively of all claims is hereby incorporated into the description by explicit reference.

According to a first aspect, the invention relates to a composition, in particular medical or pharmaceutical composition, for use in the
- treatment, in particular symptomatic treatment, of mental disorder, preferably psychosis and/or depression,
   and/or
- treatment, in particular symptomatic treatment, of drug addiction, preferably nicotine addiction and/or tetrahydrocannabinol (THC) addiction,
   and/or
- treatment, in particular symptomatic treatment, of sleep disorder
   and/or
- treatment, in particular symptomatic treatment, of pain.

The composition comprises tobacco and cannabidiol (CBD), in particular in the form of a mixture, or tobacco and cannabis, in particular in the form of a mixture. The cannabis comprises cannabidiol (CBD) as an ingredient, in particular active ingredient.

Further, the composition is prepared or used for an inhalative administration, i.e. is administered by means of inhalation.

The term "mental disorder" as used according to the present invention refers, in accordance to DSM-IV (Diagnostic and Statistical Manual of Mental Disorders (DSM)), to a psychological syndrome or pattern which is associated with distress (e.g. via a painful symptom) and/or disability (impairment in one or more important areas of functioning) and/or increased risk of death and/or which causes a significant loss of autonomy. In principle, the term "mental disorder" as used according to the present invention may refer to an acute mental disorder or a chronic mental disorder.

Preferably, the mental disorder is a disease with psychotic symptoms, i.e. a disease accompanied by psychotic symptoms. As regards possible psychotic symptoms, reference is made in its entirety to the following description.

More preferably, the mental disorder according to the present invention is selected from the group consisting of psychosis, anxiety disorder, mood disorder and bipolar disorder.

The term "psychosis" as used according to the present invention refers to an abnormal condition of the mind that results in difficulties determining what is real and what is not. Symptoms may include hallucinations, delusions, disorganization and negative symptoms such as reduced emotional expression, decreased motivation, decreased capacity of concentration, decreased capacity of attention and reduced spontaneous speech. In principle, the term "psychosis" as used according to the present invention may refer to an acute psychosis or to a chronic psychosis.

Preferably, the psychosis according to the present invention is selected from the group consisting of schizophrenia, schizophreniform disorder, schizoaffective disorder, brief psychotic disorder, delusional disorder, chronic hallucinatory psychosis and paraphrenia.

The term "schizophrenia" as used according to the present invention refers to a mental disorder which is characterized by abnormal behavior, strange speech, and a decreased ability to understand reality. Other symptoms can include false beliefs, unclear or confused thinking, hearing voices that do not exist, reduced social engagement and emotional expression, and lack of motivation. In principle, the term "schizophrenia" as used according to the present invention may refer to an acute schizophrenia or to a chronic schizophrenia.

The term "schizophreniform disorder" as used according to the present invention refers to a mental disorder which is diagnosed when symptoms of schizophrenia are present for a significant portion of the time within a one-month period, but signs of disruption are not present for the full six months required for the diagnosis of schizophrenia. The symptoms of schizophreniform disorder can include delusions, hallucinations, disorganized speech, disorganized or catatonic behavior and negative symptoms, such as an inability to feel a range of emotions (flat affects), an inability to experience pleasure (anhedonia), impaired or decreased speech (aphasia), a lack of desire for forming relationships (asociality) and a lack of motivation (avolition). While impairment in social, occupational, or academic functioning is required for the diagnosis of schizophrenia, in schizophreniform disorder an individual's level of functioning may or may not be affected. While the onset of schizophrenia is often gradual over a number of months or years, the onset of schizophreniform disorder can be relatively rapid. In principle, the term "schizophreniform disorder" as used according to the present invention may refer to an acute schizophreniform disorder or to a chronic schizophreniform disorder.

The term "schizoaffective disorder" as used according to the present invention refers to a mental disorder which is characterized by abnormal thought processes and an unstable mood. The diagnosis is made when the person has symptoms of both schizophrenia and a mood disorder - either bipolar disorder or depression - but does not meet the diagnostic criteria for schizophrenia or a mood disorder individually. In principle, the term "schizoaffective disorder" as used according to the present invention may refer to an acute schizoaffective disorder or to a chronic schizoaffective disorder.

The term "brief psychotic disorder" as used according to the present invention refers to a period of psychosis whose duration is generally shorter, is not always non-recurring, but can be, and is not caused by another condition. The disorder is characterized by a sudden onset of psychotic symptoms, which may include delusions, hallucinations, disorganized speech or behavior, or catatonic behavior. The symptoms generally last at least a day, but not more than a month, and there is an eventual return to full baseline functioning. It may occur in response to a significant stressor in one's life, or in other situations where a stressor is not apparent, including in the weeks following birth.

The term "delusional disorder" as used according to the present invention refers to a mental illness in which the patient presents delusions, but with no accompanying prominent hallucinations, thought disorder, mood disorder or significant flattening of affect. In principle, the term "delusional disorder" as used according to the present invention may refer to an acute delusional disorder or to a chronic delusional disorder.

The term "chronic hallucinatory psychosis" as used according to the present invention refers to a psychosis subtype, which is a hallucinatory case, for it is its main feature. These may be of all senses, but auditory hallucinations are the most prominent. At the beginning, the patient may realize that the hallucination is a morbid phenomenon and unaccountable. They may claim to hear a "voice" speaking, though there is no one in the flesh actually doing so. Such a state of affairs may last for years and possibly, though rarely, for life, and the subject would not be deemed insane in the ordinary sense of the word.

The term "paraphrenia" as used according to the present invention refers to a mental disorder which is characterized by an organized system of paranoid delusions with or without hallucinations and without deterioration of intellect or personality. This disorder is also distinguished from schizophrenia by a lower hereditary occurrence, less premorbid maladjustment and a slower rate of progression. Onset of symptoms generally occurs later in life, typically near the age of 60. In principle, the term "paraphrenia" as used according to the present invention may refer to an acute paraphrenia or to a chronic paraphrenia.

The term "mood disorder" as used according to the present invention refers to a group of conditions where a disturbance in the person's mood is the main underlying feature. In principle, the term "mood disorder" as used according to the present invention may refer to an acute mood disorder or to a chronic mood disorder.

The mood disorder according to the present invention can be selected from the group consisting of elevated mood such as mania or hypomania, depressed mood, preferably major depressive disorder (MDD) and bipolar disorder.

The term "mania" as used according to the present invention refers to a state of abnormally elevated arousal, affect, and energy level or "a state of heightened overall activation with enhanced affective expression together with lability of affect". The symptoms of mania include heightened mood (either euphoric or irritable), flight of ideas and pressure of speech, increased energy and decreased need for sleep and hyperactivity. Mania is also known as manic syndrome. In principle, the term "mania" as used according to the present invention may refer to an acute mania or to a chronic mania.

The term "hypomania" as used according to the present invention refers to a mood state which is characterized by persistent disinhibition and mood elevation (euphoria). It may involve irritation, but less severely than full mania. According to DSM-V criteria, hypomania is distinct from mania in that there is no significant functional impairment. Characteristic behaviors of persons experiencing hypomania are a notable decrease in the need for sleep, an overall increase in energy, unusual behaviors and actions, and a markedly distinctive increase in talkativeness and confidence, commonly exhibited with a flight of creative ideas. Other symptoms related to hypomania may include feelings of grandiosity, distractibility, and hypersexuality. In principle, the term "hypomania" as used according to the present invention may refer to an acute hypomania or to a chronic hypomania.

The term "major depressive disorder" (MDD) or "depression", as used according to the present invention refers to a mental disorder which is characterized by at least two weeks of low mood that is present across most situations. It is often accompanied by low self-esteem, loss of interest in normally enjoyable activities, low energy, and pain without a clear cause. People concerned may also occasionally have false beliefs or see or hear things that others cannot. The major depressive disorder is also commonly called clinical depression, unipolar depression or major depression. In principle, the term "major depressive disorder" and "depression", respectively as used according to the present invention may refer to an acute major depressive disorder and depression, respectively or to a chronic major depressive disorder and depression, respectively.

The term "bipolar disorder" as used according to the present invention refers to a mental disorder that causes periods of depression and periods of abnormally elevated mood. The elevated mood is significant and is known as mania or hypomania, depending on its severity, or whether symptoms of psychosis are present. During mania, an individual behaves or feels abnormally energetic, happy or irritable. Individuals often make poorly thought out decisions with little regard to the consequences. The need for sleep is usually reduced during manic phases. During periods of depression, there may be crying, a negative outlook on life, and poor eye contact with others. The risk of suicide among those with the illness is high at greater than 6% over 20 years, while self-harm occurs in 30-40%. In principle, the term "bipolar disorder" as used according to the present invention may refer to an acute bipolar disorder or to a chronic bipolar disorder.

The term "anxiety disorder" as used according to the present invention refers to a group of mental disorders which are characterized by significant feelings of anxiety and fear. Anxiety is a worry about future events, and fear is a reaction to current events. These feelings may cause physical symptoms, such as fast heart rate and shakiness. In principle, the term "anxiety disorder" as used according to the present invention may refer to an acute anxiety disorder or to a chronic anxiety disorder.

Preferably, the anxiety disorder according to the present invention is selected from the group consisting of generalized anxiety disorder, specific phobia, social anxiety disorder, separation anxiety disorder, agoraphobia, panic disorder, and selective mutism.

The term "generalized anxiety disorder" (GAD) as used according to the present invention refers to an anxiety disorder which is characterized by excessive, uncontrollable and often irrational worry about events or activities. This excessive worry often interferes with daily functioning, and sufferers are overly concerned about everyday matters such as health issues, money, death, family problems, friendship problems, interpersonal relationship problems, or work difficulties. Individuals may exhibit a variety of physical symptoms, including feeling tired, fidgeting, headaches, numbness in hands and feet, muscle tension, difficulty swallowing, vomiting, diarrhea, breathing difficulty, trembling, irritability, restlessness, sleeping difficulties, sweating and rashes. In principle, the term "generalized anxiety disorder" as used according to the present invention may refer to an acute generalized anxiety disorder or to a chronic generalized anxiety disorder.

The term "specific phobia" as used according to the present invention refers to any kind of anxiety disorder that amounts to an unreasonable or irrational fear related to exposure to specific objects or situations. As a result, the affected person tends to avoid contact with the objects or situations and, in severe cases, any mention or depiction of them. The fear can, in fact, be disabling to their daily lives. In principle, the term "specific phobia" as used according to the present invention may refer to an acute specific phobia or to a chronic specific phobia.

The term "Social anxiety disorder (SAD)" as used according to the present invention refers to an anxiety disorder which is characterized by a significant amount of fear in one or more social situations, causing considerable distress and impaired ability to function in at least some parts of daily life. These fears can be triggered by perceived or actual scrutiny from others. Individuals with social anxiety disorder fear negative evaluation from other people. Physical symptoms often include excessive blushing, excess sweating, trembling, palpitations, and nausea. Stammering may be further present, along with rapid speech. Panic attacks can also occur under intense fear and discomfort. Social anxiety disorder (SAD) is also known as social phobia. In principle, the term "social anxiety disorder" as used according to the present invention may refer to an acute social anxiety disorder or to a chronic social anxiety disorder.

The term "separation anxiety disorder (SAD)" as used according to the present invention refers to an anxiety disorder in which an individual experiences excessive anxiety regarding separation from home or from people to whom the individual has a strong emotional attachment (e.g., a parent, caregiver, significant other or siblings). In principle, the term "separation anxiety disorder" as used according to the present invention may refer to an acute separation anxiety disorder or to a chronic separation anxiety disorder.

The term "agoraphobia" as used according to the present invention refers to an anxiety disorder which is characterized by symptoms of anxiety in situations where the persons perceive their environment to be unsafe with no easy way to escape. These situations can include open spaces, public transit, shopping centers or simply being outside their home. Being in these situations may result in a panic attack. The symptoms occur nearly every time the situation is encountered and at least for more than six months. Those affected will go to great lengths to avoid these situations. In severe cases people may become completely unable to leave their homes. In principle, the term "agoraphobia" as used according to the present invention may refer to an acute agoraphobia or to a chronic agoraphobia.

The term "panic disorder" as used according to the present invention refers to an anxiety disorder characterized by reoccurring unexpected panic attacks. Panic attacks are sudden periods of intense fear that may include palpitations, sweating, shaking, shortness of breath, numbness, or a feeling that something terrible is going to happen. The maximum degree of symptoms occurs within minutes. There may be ongoing worries about having further attacks and avoidance of places where attacks have occurred in the past. In principle, the term "panic disorder" as used according to the present invention may refer to an acute panic disorder or to a chronic panic disorder.

The term "selective mutism" as used according to the present invention refers to an anxiety disorder in which a person who is normally capable of speech cannot speak in specific situations or to specific people. Selective mutism usually coexists with shyness or social anxiety. People with selective mutism stay silent even when the consequences of their silence include shame, social ostracism, or punishment. In principle, the term "selective mutism" as used according to the present invention may refer to an acute selective mutism or to a chronic selective mutism.

The term "sleep disorder" as used according to the present invention refers to a medical disorder of the sleep pattern of a person. Disruptions in sleep can be caused by a variety of issues, including teeth grinding (bruxism) and night terrors. When a person suffers from difficulty falling asleep and/or staying asleep with no obvious cause, it is referred to as insomnia. In principle, the term "sleep disorder" as used according to the present invention may refer to an acute sleep disorder or to a chronic sleep disorder.

The term "tobacco" as used according to the present invention means a product prepared from the leaves of the tobacco plant, in particular by fermenting and/or curing and/or drying and/or cutting, in particular finely cutting, them. The plant is part of the genus Nicotiana and of the Solanaceae (nightshade) family. In particular, the tobacco may be in the form of Nicotiana tabacum, Burley tobacco or in the form of a mixture of Nicotiana tabacum and Burley tobacco.

The term "Nicotiana tabacum" as used according to the present invention refers to an annually-grown herbaceous plant. It is found only in cultivation, where it is the most commonly grown of all plants in the genus Nicotiana, and its leaves are commercially grown in many countries to be processed into tobacco.

The term "Burley tobacco" as used according to the present invention refers to a light air-cured tobacco.

The term "cannabis" as used according to the present invention refers to the genus of flowering plants in the family Cannabaceae. The cannabis can be selected from the group consisting of cannabis sativa, cannabis indica, cannabis ruderalis and mixtures thereof. Preferably, the cannabis is cannabis sativa. In principle, the cannabis can be present in the composition in the form of the whole plant or in the form of a component thereof, in particular in the form of fibers of cannabis and/or in the form of seeds of cannabis and/or in the form of leaves of cannabis and/or in the form of flowers, i.e. blossoms, of cannabis. Alternatively or in combination, the cannabis can be in the form of an extract, in particular an extract of the whole plant or of a component thereof, in particular an extract of fibers of cannabis and/or an extract of seeds of cannabis and/or an extract of leaves of cannabis and/or an extract of flowers, i.e. blossoms, of cannabis. Alternatively or in combination, the cannabis can be in the form of a powder or granulate. Further, the term "cannabis" may also be denoted as "hemp" within the spirit of the present invention.

The term "cannabidiol" (CBD) as used according to the present invention refers to a compound having the below formula I:

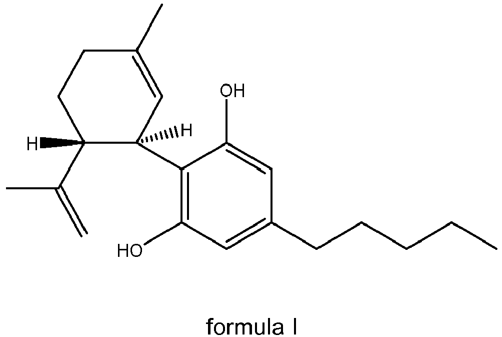

The chemical name of cannabidiol according to the nomenclature of the International Union of Pure and Applied Chemistry (IUPAC) is 2-[(1R,6R)-3-methyl-6-(1-methylethenyl)-2-cyclohexen-1 -yl]-5-pentyl-1,3-benzenediol.

Preferably, the cannabidiol (CBD) as used according to the present invention may be in the form of a natural ingredient, in particular natural active ingredient, of the cannabis.

Alternatively or in combination, the cannabidiol may be in the form of an additive of the cannabis, i.e. in the form of a compound which has been admixed to the cannabis. For this purpose, the cannabidiol may be isolated from cannabis, for example by means of extraction, or may be prepared synthetically, i.e. by means of chemical synthesis.

The term "tetrahydrocannabinol" (THC) as used according to the present invention refers to a compound having the below formula II:

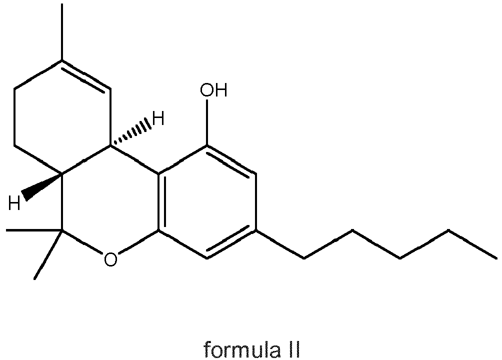

The chemical name of tetrahydrocannabinol according to the nomenclature of the International Union of Pure and Applied Chemistry (IUPAC) is (6a*R*,10a*R*)-6,6,9-Trimethyl-3-pentyl-6a,7,8,10a-tetrahydro-6*H*-benzo[c]chromen-1-ol.

The present invention rests on the surprising findings that inhalative administration of cannabidiol results in a fast and strong reduction of psychotic symptoms without or with negligible side-effects and concurrently results in better compliance, and thus less coercive (traumatizing) treatments and less rehospitalizations.

This could be successfully confirmed by the inventors within a clinical study applying cigarettes comprising cannabidiol for treating acute psychosis and chronic symptoms thereof (see example section). The psychotic symptoms, in particular depressive symptoms and negative symptoms, have been relieved upon application via inhalation.

Surprisingly, depressive symptoms have been relieved very fast, which has been measured by the Beck Depression Inventory (BDI). The Beck Depression Inventory (BDI) is the most often used self-rating instrument for depressive symptoms. Aggression has been measured via the Brøset Violence Checklist (BVC).

The better compliance of the patients was measured by the preference of cigarettes over conventional medication by the patients, who were all interested in a long-term treatment with cigarettes comprising cannabidiol. This aspect was also confirmed by cannabidiol serum concentration measurements and Subjective-Well-being under Neuroleptic Treatment Scale (SWN-K), which is a self-rating Likert scale with 20 items and good psychometric properties, when compared with outcomes in patients treated with neuroleptic drugs.

As a further advantage, the clinical study revealed that inhalative administration of cannabidiol resulted in a reduction of the number of conventional cigarettes smoked by the patients, leading to a harm reduction concerning somatic comorbidity, and thus leading to an increased life expectancy. This is relevant inasmuch as life expectancy of psychotic patients is highly impaired due to these comorbidities.

A further advantage relates to an increased trust of the patients in the treatment resulting in a better continuity in treatment (as measured by blood plasma cannabidiol concentration levels and show ups for visits as well as continuing the treatment) and a decreased number of enforced medications, a decreased number of (traumatizing) coercive measures and a decreased number of aggressive incidents of patients during acute psychiatric treatment in a case report. Thus, suicidality and suicides and somatic comorbidity and also the rate of rehospitalization can be decreased, thereby contributing to a better course of psychotic diseases if cigarettes comprising cannabidiol are used as a new treatment option.

In an embodiment of the invention, the cannabis has a ratio of cannabidiol to tetrahydrocannabinol, i.e. a cannabidiol:tetrahydrocannabinol ratio (CBD:THC ratio), from 1:1 to 25:1, in particular 2:1 to 25:1, preferably 3:1 to 20:1. The CBD:THC ratios as disclosed in this paragraph are especially useful for realizing the advantages of the present invention.

In a further embodiment of the invention, the cannabidiol has a proportion of 1% by weight to 99% by weight, in particular 3% by weight to 50% by weight, preferably 4% by weight to 21% by weight, based on the total weight of the composition.

In a further embodiment of the invention, the cannabidiol has a proportion of 1% by weight to 25% by weight, in particular 3% by weight to 23% by weight, preferably 4% by weight to 21% by weight, based on the total weight of the cannabis. The advantages of the present invention can be in particular observed using cannabidiol proportions as used in this paragraph.

In a further embodiment of the invention, the cannabis has a proportion of tetrahydrocannabinol from 1% by weight to 30% by weight, in particular 1% by weight to 20% by weight, preferably 1% by weight to 10% by weight, more preferably 1% by weight to 5% by weight, based on the total weight of the cannabis. The tetrahydrocannabinol proportions as disclosed in this paragraph are especially advantageous in terms of suppression of any undesired side effects caused by tetrahydrocannabinol.

Alternatively, the composition may be free of tetrahydrocannabinol.

Preferably, the cannabis has a propotion of 1% by weight to 99% by weight, in particular 10% by weight to 90% by weight, preferably 20% by weight to 80% by weight, based on the total weight of the composition.

In a further embodiment of the invention, the cannabis has a proportion of 50% by weight to 99% by weight, in particular 55% by weight to 90% by weight, preferably 60% by weight to 80% by weight, based on the total weight of the composition, and the mental disorder, preferably psychosis and/or depression, is an acute mental disorder, preferably an acute psychosis and/or an acute depression. Surprisingly, it turned out that the cannabis proportions as disclosed in this paragraph are especially advantageous when treating an acute mental disorder, preferably an acute psychosis and/or an acute depression.

In a further embodiment of the invention, the cannabis has a proportion of 1% by weight to 50% by weight, in particular 10% by weight to 40% by weight, preferably 20% by weight to 35% by weight, based on the total weight of the composition, and the mental disorder, preferably psychosis and/or depression, is a chronic mental disorder, preferably a chronic psychosis and/or a chronic depression. Surprisingly, it has been found that the cannabis proportions as disclosed in this paragraph are especially advantageous when treating a chronic mental disorder, preferably a chronic psychosis and/or a chronic depression.

Further, the cannabis can be a cannabis strain or a cannabis variety which is commercially available under the notation "Dancehall", "ACDC", "Suzy Q", "Ringo's Gift", "Charlotte's Web", "Remedy", "Sour Tsunami", "Hawaiian Dream", "Danceworld" or "Nordle".

In a further embodiment of the invention, the tobacco has a proportion of 1% by weight to 99% by weight, in particular 10% by weight to 90% by weight, preferably 20% by weight to 80% by weight, based on the total weight of the composition.

In a further embodiment of the invention, the composition is in the form of a smokable product, in particular in the form of a cigarette or cigar, more preferably in the form of a cigarette. In other words, in a further embodiment of the invention, the composition is administered or applied in the form of a smokable product, in particular in form of a cigarette or cigar, more preferably in the form of a cigarette.

The term "smokable product" as used according to the present invention refers to any product which is in such a way that it can be smoked by a person, in particular by a patient.

Typically, the cigarette comprises a rolling paper or cigarette paper which encloses or envelops the tobacco and cannabidiol or the tobacco and the cannabis comprising cannabidiol. The rolling paper and cigarette paper, respectively may be made from flax, cannabis (and hemp, respectively), sisal, rice, straw or esparto (halfah grass).

Further, cigarette may be in the form of a filter cigarette or filterless cigarette.

A useful smokable product according to the present invention is, for example, a cigarette comprising a mixture of cannabis and tobacco and being sold by the swiss company Heimat.

In a further embodiment of the invention, the smokable product, in particular cigarette or cigar, preferably cigarette, contains or consists of 400 mg to 800 mg of the cannabis and the mental disorder, preferably psychosis and/or depression, is an acute mental disorder, preferably an acute psychosis and/or an acute depression. Surprisingly, it has been found that cannabis amounts as disclosed in the present paragraph are especially advantageous in terms of treating an acute mental disorder, preferably acute psychosis and/or acute depression.

In a further embodiment of the invention, the smokable product, in particular cigarette or cigar, preferably cigarette, contains or consists of 8 mg to < 400 mg of the cannabis and the mental disorder, preferably psychosis and/or depression, is a chronic mental disorder, preferably a chronic psychosis and/or a chronic depression. Surprisingly, it turned out that cannabis amounts as disclosed in this paragraph are especially useful in terms of treating a chronic mental disorder, in particular a chronic psychosis and/or a chronic depression.

Further, the composition of the present invention can be administered or applied by means of a pipe, e-cigarette (electronic cigarette), bong or hookah.

If the composition, in particular smokable product, of the present invention is administered or applied by means of a hookah, the composition, in particular smokable product, preferably contains or consists of 40 mg to 800 mg, in particular 240 mg to 800 mg, of the cannabis.

If the composition, in particular smokable product, of the present invention is administered or applied by means of a bong, the composition, in particular smokable product, preferably contains or consists of 1000 mg to 2000 mg of the cannabis.

Further, the composition, in particular smokable product, may comprise 0.1% by weight to 3% by weight, in particular 0.1% by weight to 2.5% by weight, preferably 0.1% by weight to 2% by weight, of nicotine, based on the total weight of the composition, in particular smokeable product.

In a further embodiment of the invention, the psychosis is an organic psychosis, in particular a substance-induced psychosis, preferably a tetrahydrocannabinol-induced psychosis (THC-induced psychosis).

The term "organic psychosis" as used according to the present invention refers to a psychosis which is caused by organic disorders, in particular disorders of the central nervous system (such as dementia), malignancy, circulation disorder or metabolic disorder, brain injuries, autoimmune diseases or applied substances such as drugs (e.g. tetrahydrocannabinol). The organic psychosis can also be termed as "secondary psychosis" or "exogenic psychosis" according to the present invention.

The term "substance-induced psychosis" as used according to the present invention refers to a form of substance use disorder where psychosis can be attributed to substance use. It is a psychosis that results from the effects of chemicals or drugs, including those produced by the body itself. Substance-induced psychosis is commonly known as toxic psychosis.

Accordingly, the term "tetrahydrocannabinol-induced psychosis" as used according to the present invention refers to a form of substance use disorder where psychosis can be attributed to tetrahydrocannabinol (THC) use.

In a further embodiment of the invention, the psychosis is a non-organic psychosis.

The term "non-organic psychosis" as used according to the present invention refers to a psychosis which seemingly occurs without serious organic disorders. An external cause can not be determined. The non-organic psychosis can also be termed as "primary psychosis" or "endogenic psychosis" according to the present invention. The non-organic psychosis may be an acute non-organic psychosis or a chronic non-organic psychosis.

In a further embodiment of the invention, the non-organic psychosis is selected from the group consisting of schizophrenia, schizophreniform disorder, schizoaffective disorder, brief psychotic disorder, delusional disorder, chronic hallucinatory psychosis and paraphrenia.

According to a second aspect, the invention relates to a smokable product, in particular a cigarette or cigar, preferably a cigarette, for use in the
- treatment, in particular symptomatic treatment, of mental disorder, preferably psychosis and/or depression,
   and/or
- treatment, in particular symptomatic treatment, of drug addiction, preferably nicotine addiction and/or tetrahydrocannabinol (THC) addiction,
   and/or
- treatment, in particular symptomatic treatment, of sleep disorder
   and/or
- treatment, in particular symptomatic treatment, of pain.

The smokable product, in particular cigarette or cigar, preferably cigarette, comprises a composition according to the first aspect of the present invention. Alternatively, the smokable product, in particular cigarette or cigar, preferably cigarette, consists of a composition according to the first aspect of the present invention.

Typically, the cigarette comprises a rolling paper or cigarette paper which encloses or envelops the tobacco and cannabidiol or the tobacco and the cannabis comprising cannabidiol. The rolling paper and cigarette paper, respectively may be made from flax, cannabis (and hemp, respectively), sisal, rice, straw or esparto (halfah grass).

Further, the cigarette may be in the form of a filter cigarette of filterless cigarette.

With respect to further features and advantages of the smokable product, in particular with respect to the composition, reference is made in its entirety to the embodiments described under the first aspect of the invention. The features and advantages described in the context of the first aspect of the invention, in particular in terms of the composition, do apply mutatis mutandis with respect to the smokable product according to the second aspect of the invention.

Further features and advantages of the invention will become clear from the following description of preferred embodiments in form of examples in conjunction with the subject-matter of the dependent claims. The individual features can be realized either singularly or severally in combination in one embodiment of the invention. The preferred embodiments merely serve for illustration and better understanding of the invention and are not to be understood as in any way limiting the invention.

### BRIEF DESCRIPTION OF THE FIGURE

Figure 1 schematically depicts compliance of the patients measured by continuing of consumption of cigarettes as well as show up for visits.
Figure 2 schematically shows difference of Beck Depression Score in patients using CBD cigarettes versus non-CBD cigarettes. Difference is shown from start value of the intervention.
Figure 3 shows PANNS, BDI, Bröset and SWN-K Score of a case report patient, taking no drugs and refusing treatment. CBD cigarettes though did the patient accept.

### EXAMPLE SECTION

### A new treatment form of schizophrenia with cigarettes comprising cannabidiol

### 1. Methods

The study was an investigator-initiated randomized, unblinded, controlled trial that was conducted at the University Hospital of Psychiatry in Basel. The trial was financed by the University of Basel. The funders had no influence on the design or conduct of the trial and were not involved in data collection or analysis. The trial protocol, was approved by the regional scientific ethics committee for the Capital Region of Switzerland, EKNZ Nordwestschweiz. The trial was performed in accordance with the principles of the Declaration of Helsinki.

### 2. Material

For the study, a tobacco hemp cigarette has been applied which is produced by the Swiss company Heimat

### 3. Patients

26 acute voluntarily and involuntarily admitted German speaking schizophrenic inpatients were recruited for the study. Inclusion criteria were: Scoring in PANSS > 21 points, smokers, inpatients, age between 18-65 years and (Swiss) German speaking. Exclusion criteria were: non-smoker, personality disorder and/or organic psychotic disease.

### 4. Trial procedures

The participants were randomly assigned in a 1:1 ratio to either the CBD group (they were handed cigarettes comprising CBD) or the control group (they were handed cigarettes without CBD). Randomization was performed with randomizer.at.

### 5. Outcomes

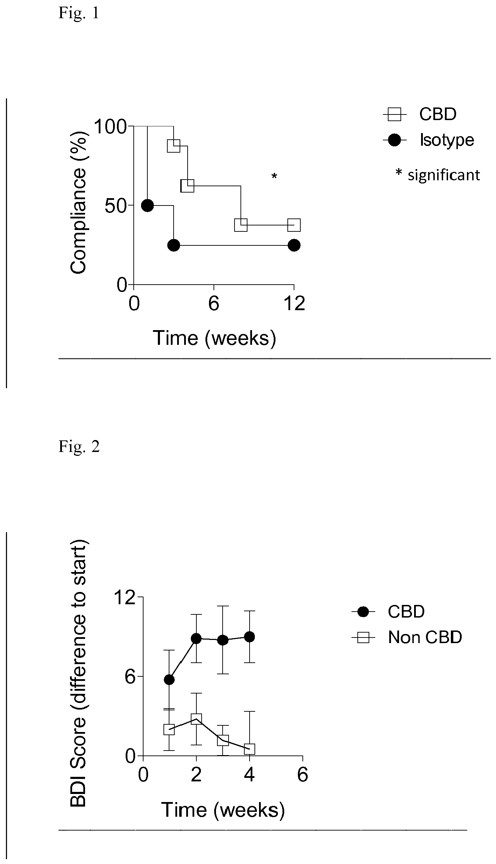

The primary study outcome was a faster remission and enhancement in terms of positive and negative psychotic symptoms (PANSS) in patients smoking CBD cigarettes when compared to smoking patients without CBD (see fig. 1). Secondary outcomes were depression (BDI), compliance, aggression (Bröset Scale), wellbeing (SWN-K), amount of smoked cigarettes, continuity in treatment and enforced medications. CBD was highly tolerable and resulted in mild side effects on a level comparable to placebo.

### 6. Results

From October 2018, to May 2019 the patients were enrolled in the study in the University Hospital in Basel. Patients were randomly assigned to the CBD group, and to the control group. Overall, the two groups were balanced with respect to baseline characteristics. In both groups, patients received additional neuroleptic treatment. The majority of patients received target doses of neuroleptic medication in accordance with the guidelines that were available at the time of the trial.

### 7. Follow-up and outcomes

The primary outcome was psychotic symptoms as measured with Positive and Negative Symptom Scale (PANSS). The PANSS is a medical scale measuring two types of schizophrenic symptoms as a gold standard that all assessments of antipsychotic behavioral disorders should follow. Positive symptoms refer to an access of normal functions, i.e. hallucinations, delusions and negative symptoms representing a loss of normal functions, i.e. anhedonia, social withdrawal, apathy.

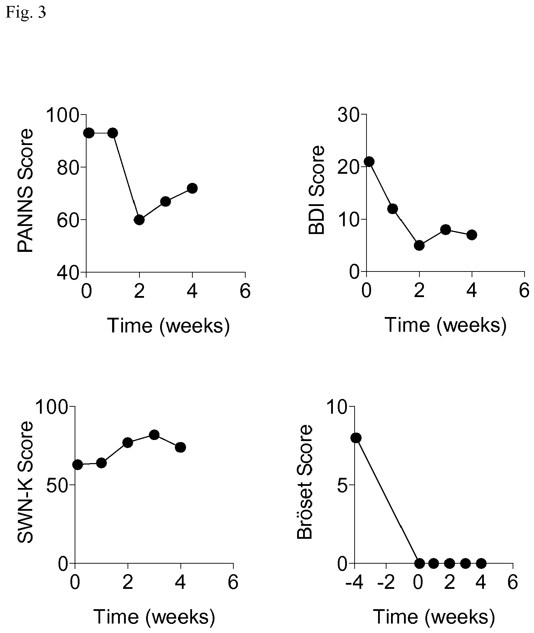

Surprisingly, depressive symptoms have been relieved very fast and very sufficient which was measured by the Beck Depression Inventory (BDI) (see fig. 3). The Beck Depression Inventory (BDI) is the most often used self-rating instrument for depressive symptoms. Aggression has been measured via the Brøset Violence Checklist (BVC). The BVC can be used by all staff working with patients or clients. The BVC when used appropriately can help to assist in preventing unwanted aggressive behavior and has been validated in Norwegian and German. The BVC assesses the presence of six observable patient behaviors namely whether the patient is confused, irritable, boisterous, verbally threatening, physically threatening, and attacking objects. The reported discriminatory ability is good with a correct prediction rate around 85%.

The better compliance of the patients (see fig. 1) was measured by adherence to the cigarette consumption, adherence to the therapists and show up at visits by the patients. Patients in the group of CBD cigarettes were mostly interested in a long-term treatment with cigarettes comprising cannabidiol. This aspect was also confirmed by cannabidiol serum concentration measurements and Subjective Well-being under Neuroleptic Treatment Scale (SWN-K), which is a self-rating Likert scale with 20 items and good psychometric properties, when compared with outcomes in patients treated with neuroleptic drugs. The median follow-up period was 3 months.

In contrast to studies with oral CBD, the effects on PANSS and especially in terms of depression were occurring faster within the present study. Additionally the patients preferred CBD cigarettes in comparison to neuroleptic drugs or drugs at all, i.e. the acceptance of the cigarettes seemed much higher and the adherence was better in patients treated with CBD cigarettes. First, symptoms have been relieved quickly upon application via inhalation, i.e. the cigarettes acted faster on psychotic symptoms than a traditional medication. Second, the use of CBD cigarettes reduced the number of cigarettes smoked leading to a significant harm reduction in the treated patients. Third, patients highly preferred cigarettes over medication. Fourth, in contrast to traditional neuroleptic drugs CBD cigarettes decreased especially depressive symptoms and negative symptoms immediately and with a stronger effect than shown in earlier studies by all other antipsychotic treatments available up to date.

In summary, the following could be successfully confirmed: CBD cigarettes are capable of reducing psychotic symptoms and depression in patients with psychotic disorders in a faster way than usual treatment. CBD cigarettes increase the adherence of patients and wellbeing in terms of their medication and treatment and it further reduces the number of enforcement measures and aggression. In patients treated with CBD cigarettes a higher rate of remission, compliance and response is observed when compared with patients smoking cigarettes without CBD.

Therefore, the application of CBD cigarettes, in particular in acute schizophrenic patients, exhibits several superior effects and advantages as compared with the use of a CBD medication and a medication with traditional neuroleptic drugs.

### Second example: Case report of a young patient suffering from schizophrenia

A 18 year old female patient came into the inpatient hospital after aggressive and desorientated behaviour in a bar. She was screaming at foreign passersby incomprehensible context. A public health officer was involved and forced the patient to an acute psychiatric war for involuntary hospitalization. Arriving at the hospital and transported by the police the patient showed agitated, aggressive behaviour. Since there was no possibility to to reach her verbally, an enforced medication using 10mg diazepame and 10mg haloperidol was needed to calm down her status display.

At first there was the suspicion of an intoxication as cause of the aggressive status display of the patient. But also after a while of hospitalization the patient showed massive impairment of formal thinking and showed mistrustfulness in several situations. As example the patient placed eye stickers at her room door on the ward to be protected against forces entering her room. Also the personal hygiene was dramandously impaired. It came up that she even got infected with Scabies ahead of the hospitalisation.

The patient showed during her hospitalisation bizarre behaviour and massive answer latencies. Also after detoxication of cocaine and THC the symptoms persisted. Initially the patient would not accept any medication.

After three weeks of hospitalization and another three times of isolation because of aggression against other patients and staff and ongoing involuntary hospitalization the patient accepted medication with olanzapine.

The medication with olanzapine was taken irregularly by the patient for two weeks. After two weeks the patient refused to proceed to take olanzapine. There was the discussion if a treatment against the wish of the patient should have been installed.

After 6 weeks of hospitalisation the patient was included into the CBD study. The amount of smoked cigarettes by the patient was between 10-20 cigarettes a day. The initial PANNS Score showed 93 points, BDI showed 21 points and SWNK showed 63 points. After ongoing consumation of CBD cigarettes PANNS and BDI Scores dropped massively (see fig. 3). After two weeks of treatment with CBD cigarettes - without parallel medication - PANNS Score dropped to 60 points and BDI dropped down to 5 points. After including of the patient into the study there was no forced medication and no isolation followed. The patient showed less aggressive behaviour, shorter answer latencies and more personal hygiene. Also there was no enforced medication, isolation or emergency medication needed. Therefore the CBD cigarettes could avoid enforcement measures for this very young patient.

## Claims

1. A composition for use in the treatment, in particular symptomatic treatment, of
- mental disorder, preferably psychosis and/or depression,
and/or
- drug addiction, preferably nicotine addiction and/or tetrahydrocannabinol addiction,
and/or
- sleep disorder
and/or
- pain,
**characterized in that** the composition comprises tobacco and cannabidiol or tobacco and cannabis comprising cannabidiol and the composition is prepared for an inhalative administration.

2. The composition according to claim 1, **characterized in that** the cannabis has a ratio of cannabidiol to tetrahydrocannabinol from 1:1 to 25:1, in particular 2:1 to 25:1, preferably 3:1 to 20:1.

3. The composition according to claim 1 or 2, **characterized in that** the cannabidiol has a proportion of 1% by weight to 99% by weight, in particular 3% by weight to 50% by weight, preferably 4% by weight to 21% by weight, based on the total weight of the composition, and/or the cannabidiol has a proportion of 1 % by weight to 25 % by weight, in particular 3 % by weight to 23 % by weight, preferably 4 % by weight to 21 % by weight, based to the total weight of the cannabis.

4. The composition according to any of the preceding claims, **characterized in that** the cannabis has a proportion of tetrahydrocannabinol from 1 % by weight to 30% by weight, in particular 1% by weight to 20% by weight, preferably 1% by weight to 10% by weight, based on the total weight of the cannabis.

5. The composition according to any of the preceding claims, **characterized in that** the cannabis has a proportion of 50 % by weight to 99 % by weight, in particular 55 % by weight to 90 % by weight, preferably 60 % by weight to 80 % by weight, based on the total weight of the composition, and the mental disorder, preferably psychosis and/or depression, is an acute mental disorder, preferably an acute psychosis and/or an acute depression.

6. The composition according to any of the claims 1 to 4, **characterized in that** the cannabis has a proportion of 1 % by weight to 50 % by weight, in particular 10 % by weight to 40 % by weight, preferably 20 % by weight to 35 % by weight, based on the total weight of the composition, and the mental disorder, preferably psychosis and/or depression, is a chronic mental disorder, preferably a chronic psychosis and/or a chronic depression.

7. The composition according to any of the preceding claims, **characterized in that** the tobacco has a proportion of 1 % by weight to 99 % by weight, in particular 10 % by weight to 90 % by weight, preferably 20 % by weight to 80 % by weight, based on the total weight of the composition.

8. The composition according to any of the preceding claims, **characterized in that** the composition is in the form of a smokable product, preferably in the form of a cigarette.

9. The composition according to any of the claims 8, **characterized in that** the smokable product, preferably cigarette, contains or consists of 400 mg to 800 mg of the cannabis and the mental disorder, preferably psychosis and/or depression, is an acute mental disorder, preferably an acute psychosis and/or an acute depression.

10. The composition according to any of the claims 8, **characterized in that** the smokable product, preferably cigarette, contains or consists of 8 mg to < 400 mg of the cannabis and the mental disorder, preferably psychosis and/or depression, is a chronic mental disorder, preferably a chronic psychosis and/or a chronic depression.

11. The composition according to any of the preceding claims, **characterized in that** the psychosis is an organic psychosis, in particular a substance-induced psychosis, preferably a tetrahydrocannabinol-induced psychosis.

12. The composition according to any of the preceding claims, **characterized in that** the psychosis is a non-organic psychosis, in particular an acute non-organic psychosis or a chronic non-organic psychosis.

13. The composition according to claim 12, **characterized in that** the non-organic psychosis is selected from the group consisting of schizophrenia, schizophreniform disorder, schizoaffective disorder, brief psychotic disorder, delusional disorder, chronic hallucinatory psychosis or paraphrenia.

14. A smokable product, in particular a cigarette, for use in the treatment, in particular symptomatic treatment, of
- mental disorder, preferably psychosis and/or depression,
and/or
- drug addiction, preferably nicotine addiction and/or tetrahydrocannabinol addiction,
and/or
- sleep disorder
and/or
- pain,
**characterized in that** the smokable product, in particular cigarette, comprises or consists of a composition according to claim 1.

15. A smokable product according to claim 14, further **characterized by** the features of the characterizing portion of any of the claims 2 to 13.
